# EUROPEAN PATENT APPLICATION

(11) **EP 3 939 618 A1**
(43) Date of publication of application: **19.01.2022**
(21) Application number: 21184896.5
(22) Date of filing: 09.07.2021
(51) Int. Cl.: A61L 2/00, F21V 7/00, A61L 2/08, A61L 9/18

(54) **METHOD FOR INACTIVATION OF A PATHOGEN COLONY IN SPACES, SURFACES AND OBJECTS USING ELECTROMAGNETIC RADIATION**

(30) Priority: 10.07.2020 IT 202000016864; 10.03.2021 IT 202100005651
(71) Applicant: Emoled Srl, 50125 Firenze (FI) (IT)
(72) Inventor: TARGETTI, Lorenzo, 50124 Firenze (IT)
(74) Representative: Biallo, Dario

(57) **Abstract**

The present invention relates to the inactivation of viruses and/or bacteria of at least 50%, or the disinfection and sanitization from bacteria and/or viruses, in spaces, surfaces or objects using an electromagnetic radiation having a wavelength comprised between 410 nm and 430 nm. In particular, it relates to a method comprising irradiating the area to be disinfected or sanitized with an electromagnetic radiation having a wavelength comprised between 410 nm and 430 nm for a time interval comprised between 5 minutes and 180 minutes, a power density comprised between 13 mW/cm² and 120 mW/cm² and an energy density comprised between 30 J/cm² and 220 J/cm², wherein the electromagnetic radiation is irradiated at a distance between the source of the electromagnetic radiation and the irradiated space, surface or object comprised from 30 centimetres to 1,5 meter.

## Description

### FIELD OF THE INVENTION

The present invention relates to the inactivation of a pathogen colony, preferably for the disinfection and sanitization, in spaces, surfaces and objects using electromagnetic radiation at a specific wavelength and power.

### BACKGROUND OF THE INVENTION

Spaces, surfaces and objects can be infected by viruses and other pathogens such as bacteria and fungi and their presence can be harmful for humans and animals.

Known disinfection and sanitization methods comprise the use of ultraviolet (UV) light or various types of chemical solutions such as alcohol, which although effective might be toxic for human and generate damages on material and biological material.

Therefore, there is a need in the art for a disinfection method allowing to sanitize a space or an object in a safe and not destructive way.

WO 2019/143647 A1 relates to illumination of light diffusing optical fibers, illumination of blue-violet light delivery systems, blue-violet light delivery systems, and methods for blue-violet light induced disinfection using the same.

EP 1 289 991 A1 relates to methods and apparatuses for treating fluids to inactivate microorganisms which may be present therein.

EP 3 517 138 A1 relates to the use of visible light, in the wavelength range of 435 to 520 nanometers, in combination with highly specific fluence and power density to create a specific light, which can reduce the growth and the number of contaminating and/or pathogenic agents on any support or in any medium.

### SUMMARY OF THE INVENTION

It has now been found that the irradiation of an area with an electromagnetic radiation at specific wavelengths and for specific time intervals causes virus inactivation and bacteria apoptosis.

It is therefore an object of the present invention a method for inactivation of bacteria and/or viruses in a space, a surface or an object of at least 50% comprising irradiating the space, surface or object with an electromagnetic radiation having a wavelength comprised between 410 nm and 430 nm for a time interval comprised between 5 minutes and 180 minutes, with a power density comprised between 13 mW/cm² and 120 mW/cm² and with an energy density comprised between 30 J/cm² and 220 J/cm².

The electromagnetic radiation is irradiated at a distance between the source of the electromagnetic radiation and the irradiated space, surface or object comprised from 30 centimetres to 1,5 meter. Preferably, the distance between the source of the electromagnetic radiation and the irradiated space, surface or object is comprised from 30 centimetres to 1 meter.

The electromagnetic radiation is a continuous electromagnetic radiation or a pulsed electromagnetic radiation. Preferably, the electromagnetic radiation is a pulsed electromagnetic radiation having a pulse repetition comprised between 10 KHz and 400 KHz.

In a particular embodiment, it is an object of the invention a method for inactivation of bacteria in a space, a surface or an object of at least 50% comprising irradiating the space, surface or object with an electromagnetic radiation having a wavelength comprised between 410 nm and 430 nm for a time interval comprised between 20 minutes and 180 minutes with a power density comprised between 13 mW/cm² and 120 mW/cm² and with an energy density comprised between 60 J/cm² and 200 J/cm².

The electromagnetic radiation is irradiated at a distance between the source of the electromagnetic radiation and the irradiated space, surface or object comprised from 30 centimetres to 1,5 meter. Preferably, the distance between the source of the electromagnetic radiation and the irradiated space, surface or object is comprised from 30 centimetres to 1 meter.

The electromagnetic radiation is a continuous electromagnetic radiation or a pulsed electromagnetic radiation. Preferably, the electromagnetic radiation is a pulsed electromagnetic radiation having a pulse repetition comprised between 10 KHz and 400 KHz.

Preferably, the above method is for the disinfection or sanitization of a space, a surface or an object from bacteria.

The object or surface does not belong to a human or animal body. That is, the method is to be applied to inanimate surfaces, objects or spaces.

In another particular embodiment, it is an object of the invention a method a method for inactivation of viruses in a space, a surface or an object of at least 50% comprising irradiating the space, surface or object with an electromagnetic radiation having a wavelength comprised between 410 nm and 430 nm for a time interval comprised between 5 minutes and 30 minutes with a power density comprised between 70mW/cm² and 120mw/cm² and with an Energy Density comprised between 30 J/cm² and 220 J/cm².

The electromagnetic radiation is irradiated at a distance between the source of the electromagnetic radiation and the irradiated space, surface or object comprised from 30 centimetres to 1,5 meter. Preferably, the distance between the source of the electromagnetic radiation and the irradiated space, surface or object is comprised from 30 centimetres to 1 meter.

The electromagnetic radiation is a continuous electromagnetic radiation or a pulsed electromagnetic radiation. Preferably, the electromagnetic radiation is a pulsed electromagnetic radiation having a pulse repetition comprised between 10 KHz and 400 KHz.

Preferably, the above method is for the disinfection or sanitization of a space, a surface or an object from viruses.

A single irradiation of electromagnetic radiation at the claimed wavelength lasting the claimed time interval, at the claimed energy and power density is enough to obtain an inactivation of at least 50% of the pathogens and more preferably to obtain disinfection or sanitization of the selected space, surface or object from bacteria and/or viruses.

In particular, a single irradiation of pulsed electromagnetic radiation at the claimed pulse repetition and wavelength lasting the claimed time interval, at the claimed energy and power density is enough to obtain an inactivation of at least 50% of the pathogens and more preferably to obtain disinfection or sanitization of the selected space, surface or object from bacteria and/or viruses.

Preferably, the irradiation of the surface, space or object by pulsed electromagnetic radiation at a wavelength comprised between 410 nm and 430 nm is performed using a LED (Light Emitting Diode). Preferably, an array of LEDs is used. Preferably, the number of LEDs in the array is comprised between 1 and 36. The electromagnetic radiation may be emitted by a LED optical fibre. In front of the LEDs, preferably a suitable optics is located in order to collimate and to even out the intensity of the resulting beam of electromagnetic radiation produced by the LEDs.

The irradiation with a power density comprised between 13 mW/cm² and 120 mW/cm² and with an energy density comprised between 30 J/cm² and 220 J/cm² means that at the level of the surface, object or space of interest, this is the power and energy density to be present.

For example, in case of a surface itself, at the level of the surface, the values of the power density and the energy density of the electromagnetic radiation are comprised between 13 mW/cm² and 120 mW/cm² and between 30 J/cm² and 220 J/cm², respectively.

In case of an object, at the level of the outer surface of the object, the values of the power density and the energy density of the electromagnetic radiation are comprised between 13 mW/cm² and 120 mW/cm² and between 30 J/cm² and 220 J/cm², respectively. However, this does not only apply to the outer surface of the object. This applies to any part of the object, also internal. The object may allow the electromagnetic radiation to pass through it (i.e. the material in which the object is made is at least transparent to the electromagnetic radiation used in the invention). Therefore, also the inside of the object can be properly irradiated and the pathogen inactivated. For example, a box has an outer surface and an inner surface. An irradiation of the box from the outside of the box may be enough to inactivate the pathogen as desired both inside and outside the box. Thus, to determine whether the pathogen is inactivated, the "surface" where energy density and power should have the claimed values is the surface defined by any desired cross section of the object.

The same is applicable to a "space". A room in which air is present is irradiated and, taken a "virtual cross section" of the volume of the room, there is inactivation of the pathogen in that specific cross section if the measured power density and energy density are within the claimed value. Thus, a given volume can be considered as subjected to inactivation if all cross sections of the volume are irradiated with an energy density and power density as claimed.

Therefore, in the following, with "surface" it is intended either a real surface or a "virtual surface" defined as a cross section above outlined.

Preferably, the radiant flux density, that is, the radiant flux received by the surface, object, or space per unit area (W/m²) remains constant during the whole irradiation. Preferably, therefore, the irradiation of the surface, space or object just takes longer (for example, a longer exposure time is selected) in case a higher radiant energy density is desired. Further, the radiant energy density, or fluence (which is the optical energy delivered per unit area), is delivered by an energy source which is so set that at the level of the surface, space or object a substantially uniform "flow" of energy is present. If the surface, space or object cannot be irradiated as a whole at the same time, for example because the area of the surface, space or object is too large, several irradiations having identical characteristics are performed, so that the whole surface, space or object is irradiated in the same way (that is, with the same electromagnetic radiation having the same wavelength, with the same power and exposure time).

Further, preferably the irradiation of the surface, space or object by the electromagnetic radiation lasts between 5 minutes and 180 minutes. The irradiation time, or "exposure time", is the time in which the irradiation takes place. More preferably, the irradiation lasts for a time interval comprised between 5 minutes and 30 minutes for the deactivation of virus and between 20 minutes and 180 minutes for the deactivation of bacteria. During this time interval, the irradiation of the surface, space or object by the blue electromagnetic radiation is continuous, that is, for a time interval between 5 -180 minutes the surface, space or object is irradiated without interruptions. The term "continuous" in case of the pulsed irradiation, means that the "interruption" in the emission of electromagnetic waves is shorter than 10% of the repetition rate of the pulsed light. Thus the exposure time above mentioned, i.e. between 5 and 180 minutes, includes the "interruptions" due to the pulsed nature of the electromagnetic radiation. For example, due to the fact that the pulsed light has a pulse comprised between 10 kHz and 400 KHz, the interruption in order not to be counted as such should not be longer than 10⁻³ seconds. In other words, the electromagnetic radiation in the present invention has to be pulsed "fast enough" to have a repetition rate above 10 kHz. In this case, the pulsed irradiation is considered as equivalent to a continuous irradiation. During the exposure time, the surface is irradiated substantially uniformly, that is, all points in the surface at the same distance from the LED receive the same radiant energy density.

### DETAILED DESCRIPTION OF THE INVENTION

According to the present invention, the term "disinfection" means that the density of the pathogen colony, be either viruses or bacteria, eventually present in the disinfected space, surface or object is reduced of a factor of at least LOG 6 after the application of the method of the invention. In particular, it means that there is an inactivation of the pathogen colony of at least 99,9999%.

According to the present invention, the term "sanitization" means that the density of the pathogen colony, be either viruses or bacteria, eventually present in the disinfected space, surface or object is reduced of a factor of at least LOG 3 after the application of the method of the invention. In particular, it means that there is an inactivation of the pathogen colony of at least 99,9%.

According to the present invention, "pathogen inactivation": means to eliminate pathogens from a surface, an object or a space. Pathogens include viruses, bacteria, and fungi.

According to the present invention, the term "time interval" means the time during which the space, surface or object from where viruses or bacteria needs to be deactivated, for example to be disinfected or sanitized, is irradiated.

According to the present invention, the term "energy density" or "fluence" means the optical energy delivered per unit area multiplied the time interval. It is intended as the total quantity of the electromagnetic radiation given at the surface.

According to the present invention, the term "power density" means the instant optical energy delivered per unit area. It is intended as the instant quantity of the electromagnetic radiation at the surface.

According to the present invention, the term "blue light" means an electromagnetic radiation having a wavelength comprised between 410 nm and 430 nm.

According to the present invention, the term "space" means a delimited two- or three-dimensional area or volume, such as a room.

According to the present invention, the term "surface" means any surface, such as the surface of a table.

According to the present invention, the term "object" means any kind of object, with any shape or function. For example, it can be an article of clothing, a shoe, an instrument, a tool.

"Space" means a confined volume, filled in general with air or any other gasses.

According to the present invention, any numerical value herein disclosed is intended said value ± 5% or ±10% of the value or within manufacturing tolerances.

According to the present invention, the term "bacteria" means any kind of bacteria. In particular, they can be bacteria of any species or strain.

According to the present invention, the term "viruses" means any kind of virus. In particular, they can be RNA or DNA viruses and they can be of any species.

According to the present invention, the electromagnetic radiation is a continuous electromagnetic radiation or a pulsed electromagnetic radiation. Preferably, the electromagnetic radiation is a pulsed electromagnetic radiation having a pulse repetition comprised between 10 KHz and 400 KHz.

It has now been found that the irradiation of a space, surface or object with an electromagnetic radiation at a specific wavelength, in particular with a pulsed electromagnetic radiation at a specific pulse repetition and wavelength, for a specific time interval and with a specific power and energy density causes virus inactivation and bacteria apoptosis.

This has been demonstrated in *in vitro* cultures of different bacterial species and viruses.

Without being bound to any particular theory, it is believed that, in bacteria, blue light is absorbed by the endogenous porphyrins contained in the bacterial cell thus initiating a photochemical reaction generating reactive oxygen species (ROS) that induce the bacteria apoptosis. In virus, it is believed that the blue light has an impact in the protein capsid damaging its integrity and therefore inactivating the virus.

The method of the invention allows to inactivate a pathogen (e.g. a virus or bacteria) of at least 50%, and preferably to disinfect or sanitize from bacteria and/or from viruses eventually present in or on a space, surface or object. Therefore, the space, surface or object can be disinfected or sanitized from any bacteria present, or from any virus present or from both viruses and bacteria which may be present in or on the space, surface or object. If not sanitized or disinfected, at least 50% of the viruses or bacteria are inactivated. The parameters of the method, i.e. time interval, power density and energy density, can be adjusted depending on the needs, in particular if inactivation of a pathogen of at least 50% is desired, if sanitization or disinfection is desired and if inactivation of bacteria or of viruses or of both is desired, based on the present disclosure and on the common general knowledge in the field.

The irradiated space, surface or object can be of any material. This means that the effects of the radiation on bacteria and/or viruses eventually present in the irradiated space, surface or object do not depend on the material receiving the radiation or on the material in or on which the bacteria and/or viruses may be present.

According to the method of the invention, the space, surface or object where the virus or bacteria may be present and need to be inactivated, disinfected or sanitized is irradiated with an electromagnetic radiation. The electromagnetic radiation has a wavelength comprised between 410 nm and 430 nm and, preferably, a pulse repetition comprised between 10 KHz and 400 KHz. Although this is technically a wavelength in the violet range, it is generally called in the field "blue light".

Preferably, the electromagnetic radiation has a wavelength comprised between 415 nm and 425 nm.

More preferably, the electromagnetic radiation has a wavelength equal to 420 nm. Being this the peak of energy absorbance by the photoreceptor contained in the bacteria (Cytochrome C), thus maximizing the selective cytotoxic effect.

According to the method of the invention, the irradiation lasts between 5 minutes and 180 minutes. During this time interval, the irradiation is continuous, that is, for a time interval between 5 minutes and 180 minutes the space, surface or object is irradiated without interruptions. For this time interval, the space, surface or object interested is irradiated preferably substantially uniformly. The specific time interval used may depend on one or more of: type of virus and/or bacteria presumably present in the irradiated space, surface or object and the required effect, i.e. inactivation of at least 50%, disinfection or sanitization.

In case of pulsed electromagnetic radiation, the fluence values between 15 and 120 J/cm² are intended as the mean of the fluence over the period of the repetition rate (i.e. number of pulses per second). Thus, "a continuous irradiation during the time interval" means that, during that time interval (or exposure time) there are no interruptions in the irradiation besides those which are intrinsic to the pulsed electromagnetic radiation's nature.

Preferably, the single time interval in which irradiation takes place is comprised between 5 minutes and 180 minutes. The irradiation takes place only once to obtain the desired effect, that is, to obtain the desired inactivation of bacteria and/or virus a single irradiation lasting between 5 min and 180 min.

In an embodiment, the method is for inactivation of at least 50%, or disinfection or sanitization from bacteria and the space, surface or object is irradiated for a time interval comprised between 20 and 180 minutes, preferably between 20 minutes and 60 minutes.

In another embodiment, the method is for inactivation of at least 50%, or disinfection or sanitization from viruses and the space, surface or object is irradiated for a time interval comprised between 5 minutes and 30 minutes, preferably between 15 minutes and 30 minutes.

The radiation is delivered by an energy source which is so set that at the level of the space, surface or object to be disinfected or sanitized a substantially uniform flow of energy is present.

Preferably, the emission power density while irradiating is comprised between 70 mW/cm² and 120 mW/cm². This power density range allows to trigger the targeted biological reactions in bacteria and viruses.

Preferably, the energy density of the electromagnetic radiation over the treated space, surface or object is uniform. This means that all portions or parts of the treated space, surface or object receive this energy density. If the space, surface or object is too big to be treated in a single treatment, the space, surface or object can be divided in portions and each portion may undergo the treatment according to the method of the invention till the whole space, surface or object receives the electromagnetic radiation for the claimed time interval and at the proper power and energy density. In case of an object, its outer surface is receiving the radiation at this power and energy density. The material in which the object is made between its outer surfaces may not receive the radiation at such power or energy density.

In an embodiment, the method is for inactivation of at least 50%, disinfection or sanitization from bacteria and it comprises the application of an electromagnetic radiation, in particular of a pulsed electromagnetic radiation having a pulse repetition comprised between 10 KHz and 400 KHz, having the specified wavelength for a time interval comprised between 20 minutes and 180 minutes, preferably between 20 and 60 minutes, and with an energy density comprised between 61 J/cm² and 200 J/cm² at the surface level. This means that the irradiated outer surface of the object receives the radiation at said energy density. In this embodiment power density is comprised between 13 mW/cm² and 120 mW/cm², preferably between 18 and 60 mW/cm², more preferably the power density is 18 mW/cm², 52 mW/cm², 55 mW/cm² or 120 mW/cm². This means that the irradiated outer surface of the object receives the radiation at said power densities. In this embodiment, preferably, the energy density is comprised between 140 J/cm² and 185 J/cm², more preferably the energy density is 144 J/cm², 185 J/cm² or 200 J/cm².

In a preferred embodiment, the time interval in which irradiation takes place is equal to 180 minutes at a power density equal to 18 mW/cm² and at an energy density equal to 200 J/cm².

In a preferred embodiment, the time interval in which irradiation takes place is equal to 60 minutes at a power density equal to 51 mW/cm² and at an energy density equal to 185 J/cm².

In an embodiment, the method is for disinfection or sanitization from viruses and it comprises the application of the electromagnetic radiation, in particular of the pulsed electromagnetic radiation having a pulse repetition comprised between 10 KHz and 400 KHz, having the specified wavelength for a time interval comprised between 5 minutes and 30 minutes, preferably comprised between 15 and 30 minutes, and with an energy density comprised between 36 J/cm² and 216 J/cm² at the surface level. This means that the irradiated outer surface of the object receives the radiation at said energy density. In this embodiment power density is comprised between 70 mW/cm² and 120 mW/cm². This means that the irradiated outer surface of the object receives the radiation at said power density.

In this embodiment, preferably the energy density is comprised between 108 J/cm² and 216 J/cm².

In this embodiment, the time interval in which irradiation takes place is preferably equal to 15 minutes or 30 minutes.

In this embodiment, the power density of the electromagnetic radiation is preferably equal to 70 mW/cm² or 120 mW/cm².

In preferred embodiments, the time interval in which irradiation takes place is comprised between 15 minutes and 30 minutes, the energy density is comprised between 108 J/cm² and 216 J/cm² and the power density is comprised between 70 mW/cm² and 120 mW/cm².

In preferred embodiments:
the time interval in which irradiation takes place is preferably equal to 30 minutes and the power density and energy density of the electromagnetic radiation are preferably equal to 120 mW/cm² and 210 J/cm², respectively;
the time interval in which irradiation takes place is preferably equal to 30 minutes and the power density and energy density of the electromagnetic radiation are preferably equal to 70 mW/cm² and 126 J/cm², respectively;
the time interval in which irradiation takes place is preferably equal to 15 minutes and the power density and energy density of the electromagnetic radiation are preferably equal to 120 mW/cm² and 108 J/cm², respectively.

In some exemplary embodiments, the parameters combination disclosed in the following Table 1 can be used in the method of the invention for inactivating of at least 50%, disinfecting or sanitizing the irradiated space, surface or object from bacteria of one or more species selected from the group consisting of *Escherichia Coli, Staphilococcus Aureus* and *Pseudomonas Aeruginosa.*

**Table 1. Exemplary method parameters for bacteria.**

| Bacteria Type | power density (mW/cm²) | Time (minutes) | energy density (J/cm²) |
|---|---|---|---|
| Escherichia Coli | 18 | 180 | 200 |
| | 17 | 180 | 185 |
| | 51 | 60 | 185 |
| Staphilococcus Aureus | 18 | 180 | 200 |
| | 17 | 180 | 185 |
| Pseudomonas Aeuroginosa | 17 | 180 | 185 |
| | 51 | 60 | 185 |
| | 120 | 20 | 144 |

In some exemplary embodiments, the parameters combinations disclosed in the following Table 2 can be used in the method of the invention for inactivating of at least 50%, disinfecting or sanitizing an irradiated space, surface or object from viruses belonging to one or more virus families selected from the group consisting of: adenovirus, Respiratory Syncytial Virus and coronavirus. The viruses can be of any specie or serotype within the mentioned families. For example, the virus can be a coronavirus of the strain severe acute respiratory syndrome coronavirus 2 (SARS-CoV-2), causing the coronavirus disease 19 (COVID-19).

**Table 2. Exemplary method parameters for viruses.**

| Virus Type | Power density (mW/cm²) | Time (minutes) | Energy density (J/cm²) |
|---|---|---|---|
| Adenovirus | 120 | 30 | 216 |
| | 70 | 30 | 126 |
| Respiratory Syncytial Virus | 120 | 30 | 216 |
| SARS-CoV-2 (COVID-19) | 120 | 30 | 216 |
| | 120 | 15 | 108 |

Preferably, the electromagnetic radiation is irradiated at a distance between the source of the electromagnetic radiation and the space, surface or object to be irradiated comprised from 30 centimetres to 1,5 meter, more preferably from 30 centimetres to 1 meter. The distance may be calculated and maintained by an infrared proximity sensor measuring the distance from target (i.e. surface or object or space).

This feature allows the commercial, public, and working environments to be properly and uniformly irradiated without preventing their access and usability. Preferably, the irradiation can be performed by at least an apparatus which can be installed next to general light and avoiding that the activation of the method prevents the use of the space.

Preferably, the maximum irradiated area at the same time is 2.500 cm². The irradiated area, that is, the area irradiated at the same time with the electromagnetic radiation, is preferably a circle. Preferably, the maximum diameter of the circle is 60 centimetres, more preferably 56 centimetres.

As mentioned above, a single irradiation suffices to obtain inactivation of at least 50%, disinfection or sanitization of the selected space, surface or object from bacteria and/or viruses.

Preferably, the repetition rate of the pulsed electromagnetic radiation is equal to 400 KHz.

Preferably the irradiation is performed using a LED. Preferably, it is performed using an array of LEDs. Preferably the number of LEDs is comprised between 1 and 36. Preferably LED's are enclosed in a combination of reflectors and lenses in order to collimate and to even out the intensity of the resulting beam.

In particular, the one or more LEDs are adapted to emit a beam of electromagnetic radiations having a wavelength comprised between 410 nm and 430 nm and they have a suitable optics to collimate the beam of electromagnetic radiation and render it uniform in intensity over a cross section of the beam. The beam generated by the LEDs can exit the optics via a suitable opening. The maximum area that the LEDs can irradiate at the same time is equal to 2500 cm².

The LED type is preferably LHUV-0415-A070, from Luxeon.

The irradiation can be performed preferably using different types of apparatuses, for example each apparatus having one or more LEDs which emits at the desired radiation and having the necessary power. The operator irradiates the surface for the chosen time interval through the type of apparatus optimized for his needs.

The apparatuses may be portable, free standing, table standing or whole case enclosures. The optics of the apparatus is such that the irradiation on the area of interest is substantially uniform.

In an exemplary embodiment, the optics of the apparatus is modular. Each module includes a panel which can be assembled preferably in multiples of 1 to 8. Each panel can have more optics 8(i.e. LEDs and focusing lenses) and each optic can comprise a number of LEDs preferably comprised between 1 and 36.

One or more panels can be assembled together to form the apparatuses of different types above described.

The apparatuses are of substantially three types: apparatuses for sanitation of spaces and surfaces; portable apparatuses; apparatuses for sanitation of objects.

The invention will be now described in detail in some of its embodiments with no-limiting reference to the appended drawings:
- Fig. 1 is a schematic front view of a panel including the optics for the application of the method according to the invention;
- Figs. 2 and 3 are schematic front views of two different embodiments of the panel of Fig. 1;
- Figs. 4 and 5 are schematic perspective views of two different embodiments of a table-standing device including the panel of Figs. 1-3;
- Fig. 6 represents schematic perspective views of a free-standing device including the panel of Figs. 1-3;
- Figs. 7 and 8 are schematic perspective views of a ceiling-mounted device including the panel of Figs. 1-3;
- Figs. 9 and 10 are schematic perspective views of two different embodiments of a portable device including the panel of Figs. 1-3;
- Figs. 11 and 12 are schematic perspective views of two different embodiments of a box device including the panel of Figs. 1-3.
- Figs. 13 and 14 are schematic perspective views of two different embodiments of a piece of furniture including the panel of Figs. 1-3.

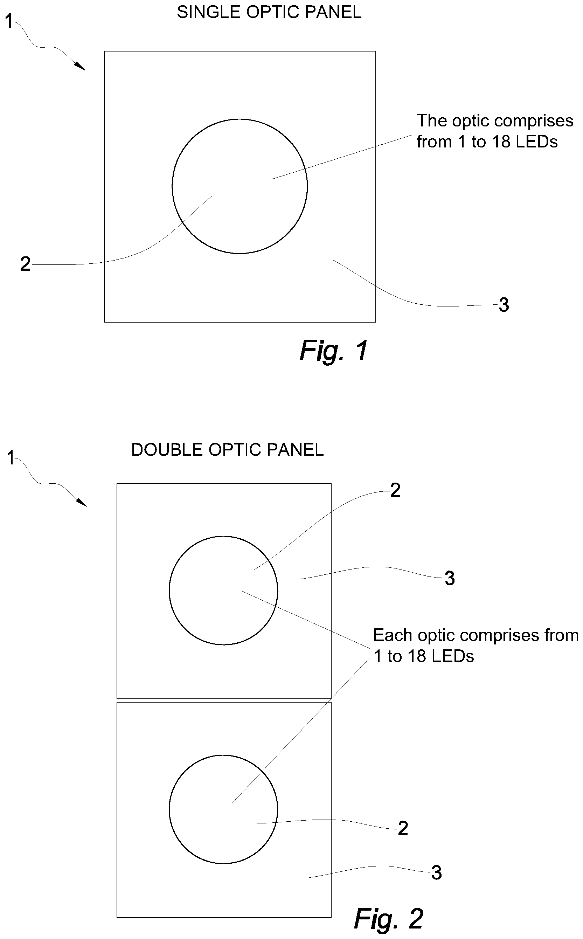

With initial reference to fig. 1, a panel including the one or more optics to be used in an apparatus operating according to the method of the present invention is represented and indicated with reference number 1.

With reference to fig. 1, the panel 1 includes an optical element 2 for the application of the method according to the present invention. The panel 1 further includes a supporting element 3 which supports the optical element 2. Preferably, the supporting element 3 is substantially flat, that is, one of its dimensions, for example its thickness, is smaller than its width and length. The supporting element 3 may have a square cross section. It is evident that, in other embodiments, the supporting structure3 may have a different shape such as for example a circular shape.

The optical element 2 is configured to perform the irradiation for the application of the method according to the present invention. Accordingly, the optical element 2 emits electromagnetic radiation, in particular pulsed electromagnetic radiation, at the desired blue wavelength and with the necessary power and energy density. Advantageously, the optical element 2 comprises one or more LEDs. Further advantageously, the optical element 2 comprises between 1 and 36 LEDs.

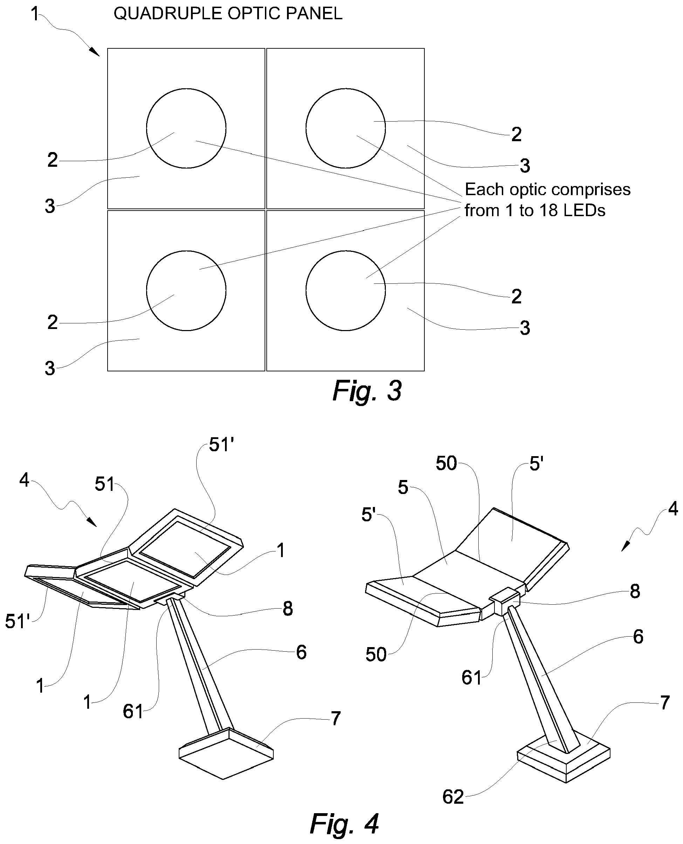

In the embodiment depicted in figures 2 - 3, the panel 1 has a modular design which allows to arrange a plurality of supporting elements 3 adjacent one each other to form a single structure. In this embodiment, each supporting element 3 supports a corresponding optical element 2. Preferably, the elements 3 of the plurality of supporting elements 3 have a square cross section defining four sides. Two supporting elements 3 are connected to each other by their respective sides, that is, they are joined along the sides of their square cross section. The panels 1 are connected so that they form a planar overall structure. In one embodiment, two supporting elements 3 are structurally and electrically connected to each other. Advantageously, a frame is provided to structurally connect two supporting elements 3, as detailed below with reference to figures 4-8. Further advantageously, the supporting elements 3 are inserted in the frame.

The supporting elements 3 on the side opposite to the optical elements 2 may be attached to the frame, for example by means of glue and/or adhesive tape and/or screws and/or other removable or non-removable coupling means. The frame has an opening so that the optical elements 2 are capable to irradiate a space, surface, or object with electromagnetic radiation. It is evident that, in one embodiment, the opening through the frame may be replaced by a surface made of a material which is transparent to the electromagnetic radiation emitted by the optical elements 2. Preferably, electrical wiring (not shown) is provided to electrically connect two supporting elements 3 and/or two optical elements 2. Further preferably, the electrical wiring may connect the panel 1 to a power supply source (not shown).

For example, with now reference to fig. 2, two connecting elements 3 can be arranged adjacent one each other in order to form a double panel. Each of the two supporting elements 3 supports a corresponding optical element 2. Therefore, the double panel 1 comprises two optical elements 2. Advantageously, the double panel 1 is substantially flat and elongated in one direction. The two supporting elements 3 are arranged substantially coplanar.

Further, with now reference to fig. 3, four supporting elements 3 can be arranged adjacent one each other in order to form a panel 1 which is quadruple. The quadruple panel 1 comprises four optical elements 2. Preferably, the quadruple panel 1 is substantially flat and defines a square cross section. In one embodiment, the panels 1 are equally spaced one each other.

Hereinafter, the term "panel" is used to indicate a panel 1 according to any of the above-mentioned embodiments, including a single panel or a multiple panel such as for example a double panel or a quadruple panel. However, it is evident that in other embodiments, the panel 1 can comprise different combinations of optical elements 2, for example it can comprise three, five, six, seven, eight or more optical elements 2.

One or more panels 1 according to any of the above-mentioned embodiments can be used for the application of the method according to the present invention in a set of different instruments either portable, free standing, table standing or whole case enclosures, as detailed below.

With now reference to fig. 4, an apparatus 4 for the irradiation of a space, surface or object according to the method of the invention is depicted. The apparatus 4 is a table-standing device. The apparatus includes three panels 1. Each panel 1 includes a frame 5, 5' in which it is inserted. Each frame 5, 5' has an opening 51, 51' so that the optical elements 2 are capable to irradiate the space, surface, or object with electromagnetic radiation. It is evident that, in one embodiment, the openings 51, 51' through the frames 5, 5' may be replaced by a surface made of a material which is transparent to the electromagnetic radiation emitted by the panels 1. Advantageously, the table-standing device 4 is configured to disinfect or sanitize an object located in front of the optical elements located in the panels 1. In the embodiment depicted in figure 4, the frames 5, 5' are arranged adjacent one each other. Preferably, the frames 5, 5' have a geometry which is similar to the geometry of the panel 1 which they contain. The frames are substantially flat. A cross section of the frames is preferably square. Each frame may be considered substantially planar. Further preferably, the frames 5, 5' are arranged in a series with a central frame 5 and two lateral frames 5'. The connection among the central frame 5 and the lateral frames 5' takes place at the sides of the frames. In other words, each frame defines four sides, divided in two couples of opposite sides. The lateral frames are fixed to a couple of opposite sides of the central frame, a lateral frame per side. As shown in figure 4, the lateral frames 5' are inclined with respect to the central frame 5 in a way that the radiation of the relevant panels 1 can span a larger area of a surface opposite the panels 1. The central frame 5 may define a central plane, for example parallel to the surface to be irradiated. The lateral frames 5' define two lateral planes, each forming an angle with the central plane. The two lateral frames 5' are connected to the central frame 5 in such a way that they form a symmetric structure with respect to a plane parallel to two sides of the central frame 5 and dividing the central frame in two identical parts. Advantageously, it is possible to adjust the inclination of the lateral frames 5' with respect to the central frame 5. In one embodiment, a frame joint 50 is interposed between each of the lateral frames 5' and each of two opposite sides of the central frame 5. The frame joints 50 may be compliant joints or hinge joints or any other type of joints. The frame joints 50 allow to adjust the inclination of the lateral frames 5' with respect to the central frame 5 along two opposite sides of the central frame 5. Preferably, each frame joint 50 allows to adjust the angle between the central plane defined by the central frame 5 and the lateral plane defined by each lateral frame 5' in a range comprised between 180° (in this condition the lateral plane and the central plane are parallel and/or coplanar) and at least 90° (in this condition the lateral plane and the central plane are perpendicular to each other). The apparatus 4 further includes an arm 6 and a base support 7. The arm 6 has a first and a second axially opposed ends 61, 62 and connects the connected frames 5, 5' described above to the base support 7. More in detail, the first end 61 of the arm 6 is attached to the connected frames 5, 5' and the second end 62 of the arm 6 is attached to the base support 7. The base support 7 is configured to support the apparatus 4 on a support surface (not shown) such as for example the surface of a table or other piece of furniture. The panels 1 may be oriented in a way that their radiation points towards the support surface. Advantageously, the base support 7 is relatively heavy in order to assure the stability of the apparatus 4 on the support surface. In one embodiment, the arm 6 extends along a straight line. More preferably, it defines a single straight direction. Preferably, the arm 6 is relatively rigid. In one embodiment, the first end of the arm 6 is attached to the central frame 5. Preferably, an arm joint 8 is interposed between the first end of the arm 6 and the central frame 5 in order to allow an adjustment of the inclination of the frames 5, 5' with respect to the arm 6. It is evident that, in other embodiments, the apparatus 4 may comprise any number and any type of panels 1, not only three as depicted in figure 4.

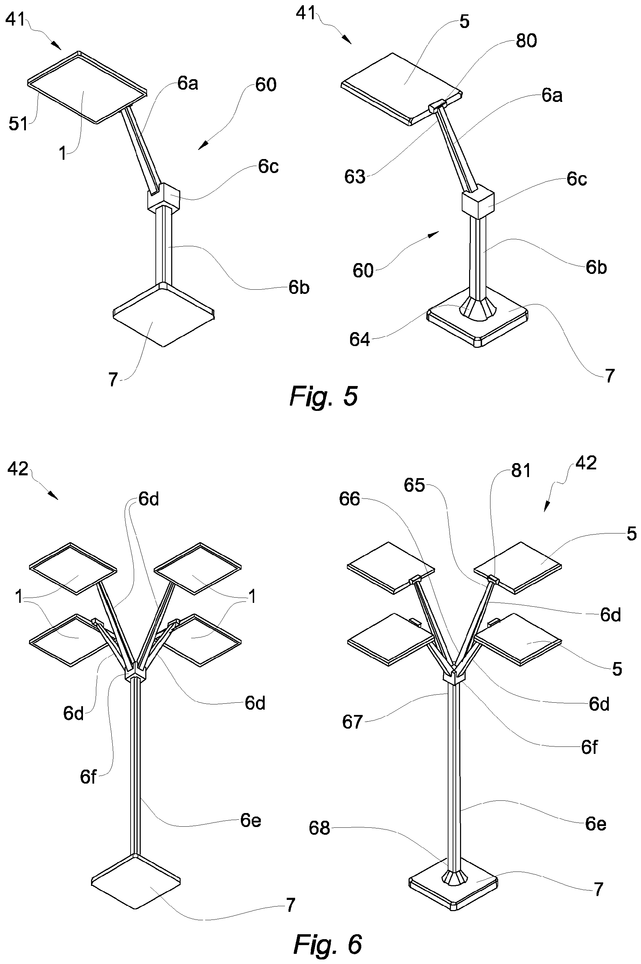

Further, with now reference to fig. 5, the apparatus 41 according to another embodiment includes a single panel 1 framed by a single frame 5. The frame 5 is then connected to a base support 7 identical to the base support of the embodiment in figure 4. The base support 7 and the frame are connected by means of an arm 60.The arm 60 extends between a first end 63 of the arm 60 and a second end 64 of the arm 60. The first end 63 is attached to the frame 5 and the second end 64 is attached to the base support 7. In one embodiment, an arm joint 80 is interposed between the first end 63 of the arm 60 and the frame 5 in order to allow an adjustment of the inclination of the frame 5 with respect to the arm 60. Advantageously, the arm 60 includes a first arm segment 6a, a second arm segment 6b, and a arm segment joint 6c interposed between the first arm segment 6a and the second arm segment 6b. The first arm segment 6a links the frame 5 to the arm segment joint 6c, while the second arm segment 6b links the arm segment joint 6c to the base support 7. Preferably, each of the arm segments 6a and 6b extends along a straight line and further preferably the first arm segment 6a is inclined with respect to the second arm segment 6b. In one embodiment, the arm segment joint 6c allows to adjust the inclination of the first arm segment 6a with respect to the second arm segment 6b. Preferably, the inclination of the first arm segment 6a with respect to the second arm segment 6b may be adjustable between a first position where the first arm segment 6a is perpendicular to the second arm segment 6b and a second position where the first arm segment 6a is parallel to the second arm segment 6b. It is evident that the length of the first and second arm segments 6a and 6b may be different according to different embodiments. In particular, the apparatus 4 according to one embodiment may be a free-standing device which is configured to stand for example on a floor. With respect to the table-standing device, in the case of a free-standing device the arm 60 may be longer and the base support 7 may be larger and/or heavier.

Further, with now reference to fig. 6, the apparatus 42 according to another embodiment includes a plurality of panels 1, each panel 1 being framed by a single frame 5 and each frame 5 being supported by a dedicated first arm segment 6d. The apparatus 42 is a free-standing device. Advantageously, the apparatus 42 is configured to disinfect or sanitize a space, such as a room, where the apparatus 42 is standing. In one embodiment, the apparatus 42 is provided with four panels 1, four frames 5 and four first arm segments 6d equally spaced one each other. Preferably, each of the first arm segments 6d extends along a straight line between a first and a second axially opposed ends 65 and 66. Advantageously, an arm joint 81 is interposed between each frame 5 and the first end 65 of the relevant first arm segment 6d in order to allow the adjustment of the inclination between the frame 5 and the relevant first arm segment 6d. The first arm segments 6d are connected to a base support 7 by means of a second arm segment 6e. The second arm segment 6e extends along a straight line between a first and a second axially opposed ends 67 and 68. In one embodiment, an arm segment joint 6f is interposed between the first end 67 of the second arm segment 6e and the second end 66 of each of the four first arm segments 6d. Advantageously, the arm segment joint 6f allows to adjust the inclination of each of the first arm segments 6d with respect to the second arm segment 6e. Further advantageously, the inclination of each of the first arm segments 6d with respect to the second arm segment 6e may be individually adjustable between a first position where the first arm segment 6d is perpendicular to the second arm segment 6e and a second position where the first arm segment 6d is parallel to the second arm segment 6e.

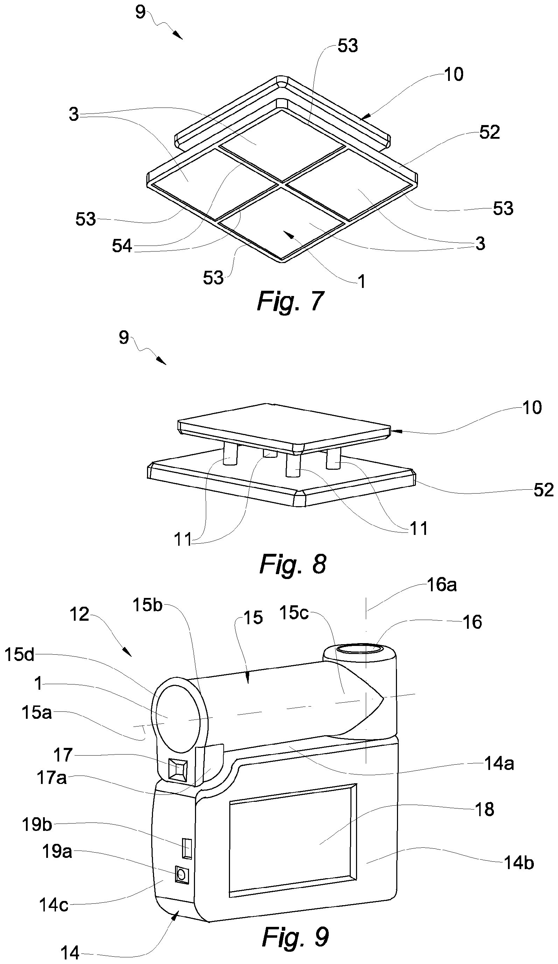

Further, with now reference to figs. 7 and 8, an apparatus 9 is a ceiling-mounted device which comprises the quadruple panel 1 as described with reference to fig. 3. Advantageously, the apparatus 9 is configured to disinfect or sanitize a space, such as a room, where the apparatus 9 is mounted. The quadruple panel 1 is hold in place by a frame 52. Preferably, the frame 52 is substantially flat, that is, one of its dimensions, for example its thickness, is smaller than its width and length. Further preferably, the frame 52 has a square cross section. In one embodiment, the frame 52 defines four sides, divided in two couples of opposite parallel sides. Advantageously, the four supporting elements 3 of the quadruple panel 1 are separated by partition walls 54 which are parallel to the four sides of the frame 52. The frame 52 has four openings 53 so that the optical elements (not visible) of the quadruple panel 1 are capable to irradiate the space with electromagnetic radiation. Preferably, the four openings 53 are separated by the partition walls 54. It is evident that, in one embodiment, the openings 53 through the frame 52 may be replaced by a surface made of a material which is transparent to the electromagnetic radiation emitted by the quadruple panel 1. The apparatus 9 further comprises a ceiling mount bracket 10 which is configured to removably mount the device 9 to a ceiling in a way that the bracket 10 points toward the ceiling and the quadruple panel 1 points in a direction opposite to the ceiling and preferably points toward the ground or floor. Advantageously, the device 9 is configured to be mounted to a ceiling in a way that the bracket 10 and/or the frame 52 and/or the quadruple panel 1 are substantially parallel to the ceiling and/or to the ground. The frame 52 defines a first side of the frame 52 where the panel 1 is inserted and a second side of the frame 52 opposite to the first side of the frame 52. In addition, the bracket 10 defines a first side of the bracket 10 which is configured to be mounted to a ceiling and a second side of the bracket 10 opposite to the first side of the bracket 10. Preferably, one or more linking elements 11 are provided to link the second side of the bracket 10 to the second side of the frame 52. In one embodiment, the number of linking elements 11 is four. Each of the linking elements 11 is an overhanging member that projects from the second side of the bracket 10 toward the second side of the frame 52. Preferably, the linking elements 11 extend along a straight line and are equally spaced one each other. It is evident that, in other embodiments, the quadruple panel 1 can be replaced by any number and any type of panel 1.

Further, with now reference to fig. 9, an apparatus 12 for the irradiation of a space, surface or object according to the method of the invention is depicted. The apparatus 12 is a portable device. The apparatus 12 is equipped with a main body 14 and an optical head 15 linked to the main body 14. The main body 14 is a parallelepiped with rounded edges wherein a top wall 14a, a front wall 14b and a side wall 14c are defined. The optical head 15 extends along an optical head axis 15a between a first and a second axially opposed ends 15b and 15c. The optical head 15 has a circular cross section. At the first end 15b of the optical head 15 an opening 15d and a panel 1 are provided in a way that the panel 1 is capable to irradiate the space, surface or object with electromagnetic radiation through the opening 15d. It is evident that, in one embodiment, the opening 15d may be replaced by a surface made of a material which is transparent to the electromagnetic radiation emitted by the panel 1. Preferably, the panel 1 and the opening 15d have a circular cross section. The panel 1 may be perpendicular to the optical head axis 15a. In one embodiment, the optical head 15 is adjustable with respect to the main body 14 by means of an optical head hinge 16 between the main body 14 and the optical head 15. Preferably, the optical head hinge 16 is rotatable along an optical head hinge axis 16a which projects from the main body 14. In one embodiment, the optical head axis 15a is perpendicular to the optical head hinge axis 16a. Advantageously, the optical head 15 is rotatable about the optical head hinge 16 between a rest position wherein the optical head 15 is parallel and adjacent to the top wall 14a of the main body 14 in a way that the size of the apparatus 12 is minimized and a work position (not shown) wherein the optical head 15 is rotated towards the space, surface or object to be irradiated with electromagnetic radiation. Preferably, the optical head 15 is further provided with a distance indicator sensor 17 for correctly positioning the optical head 15 over a target surface. The distance indicator sensor 17 is located at the first end 15b of the optical head 15, next to the panel 1. A sensor support 17a may be provided at the first end 15b of the optical head 15 to support the distance indicator sensor 17. Preferably, the sensor support 17a projects from the first end 15b of the optical head 15 in a direction perpendicular to the optical head axis 15a. In addition, a touch screen display 18 may be provided for setting and managing the optimal functioning of the device 13. The touch screen display 18 is visible on the front wall 14b of the main body 14. The touch screen display 18 is substantially flat and has a rectangular shape. The touch screen display 18 is parallel to the front wall 14b. In one embodiment, the apparatus 12 has a battery power supply (not visible) with a power supply input 19 for charging the battery. The battery power supply may be located inside the main body 14, whereas the power supply input 19 is located on the side wall 14c of the main body 14. Preferably, a port 19b is provided on the side wall 14c of the main body 14 to serve as an interface between the apparatus 12 and a computer or other devices (not shown). In one embodiment, the port 19b may be used in order to configure the apparatus 12 to perform the method according to the present invention.

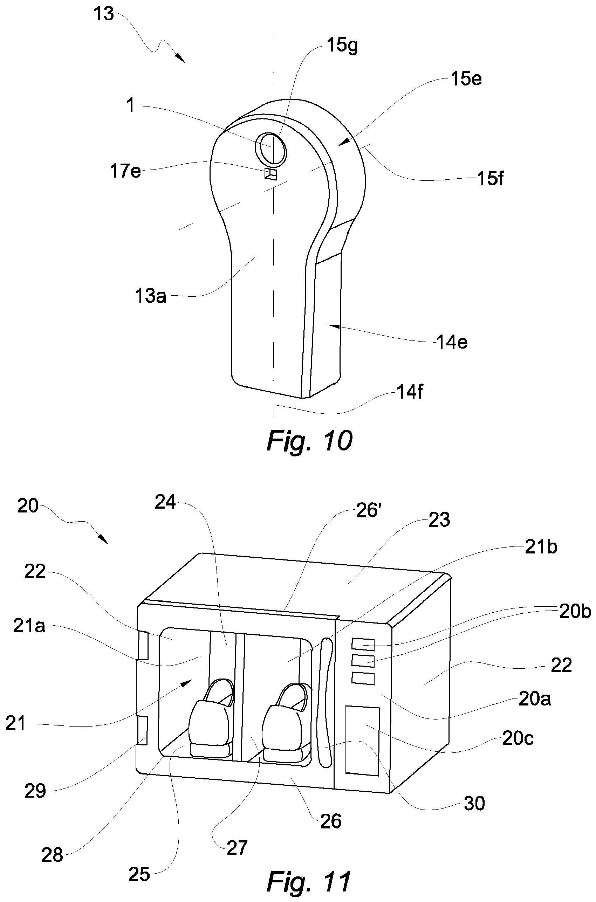

Further, with now reference to fig. 10, an apparatus 13 for the irradiation of a space, surface or object according to the method of the invention is depicted. The apparatus 13 is a portable device. The apparatus 13 has a main body 14e and an optical head 15e integral with the main body 14e. The main body 14e extends along a straight line 14f and has a rectangular cross section. Preferably, the main body 14e may serve as a handle to manoeuvre the optical head 15e. The optical head 15e extends along an optical head axis 15f which is perpendicular to the straight line 14f of the main body 14e. The optical head 15e has a circular cross section. Advantageously, the main body 14e and the optical head 15e define a front wall 13a of the apparatus 13. The front wall 13a is substantially flat. The optical head 15e is provided with an opening 15g through the front wall 13a. Preferably, the opening 15g is circular. A panel 1 is provided in the optical head 15e. The panel 1 is capable to irradiate the space, surface or object with electromagnetic radiation through the opening 15e. Advantageously, the optical head 15e comprises a distance indicator sensor 17e for correctly positioning the optical head 15e over a target surface. The distance indicator sensor 17e is located at the front wall 13a next to the panel 1.

Further, with now reference to fig. 11, an apparatus 20 for the irradiation of a space, surface or object according to the method of the invention is depicted. The apparatus 20 is a box device. The apparatus 20 comprises a chamber 21 configured to receive an object such as for example a shoe or a pair of shoes, and at least one panel 1 (not visible) configured to irradiate the object inside the chamber 21 and/or a surface of the object inside the chamber 21 and/or the space inside the chamber 21. Advantageously, the apparatus 20 is configured to disinfect or sanitize the object inserted into the chamber 21. In one embodiment, the apparatus 20 has a box enclosure structure and the chamber 21 is defined by a plurality of walls 22, 23, 24, 25 of said box enclosure structure. Preferably, the walls of the plurality of walls 22, 23, 24, 25 are substantially flat and define a parallelepiped shape of the box enclosure structure and of the chamber 21. In particular, the box enclosure structure includes a front wall 20a, two opposite side walls 22, a top wall 23, a back wall 24, and a bottom wall 25. The panel 1 may be mounted to the top wall 23 of the chamber 21 in a way that the optical element of the panel 1 emits the electromagnetic radiation toward the bottom wall 25 of the chamber 1. Preferably, the box enclosure structure further includes a front opening 26' through the front wall 20a for inserting the object into the chamber 21 and for extracting the object from inside the chamber 21. Further preferably, the box enclosure structure includes a front door 26 to close the front opening 26'. The front door 26 is coplanar with the front wall 20a. In one embodiment, the chamber 21 is split into two sub-chambers 21a, 21b by a partition wall 27 located inside the chamber 21. In one embodiment, the partition wall 27 is parallel to the side walls 22. Each of the two sub-chambers 21a, 21b has a parallelepiped shape. Advantageously, each sub-chamber 21a, 21b is equipped with at least one panel 1 (not visible) configured to irradiate the respective chamber 21a, 21b. Further advantageously, each sub-chamber 21a, 21b is configured to receive an object through the front opening 26', and irradiate the object or a surface of the object inside the sub-chamber 21a or 21b separately from the other sub-chamber 21b or 21a, respectively. In one embodiment, the front door 26 has a front window 28 made of a material which is transparent to visible electromagnetic radiation to allow a user to monitor the chamber 21 from the outside. The front window 28 is substantially flat and coplanar with the front door 26. Preferably, the front door 26 is articulated to the box enclosure structure by means of a door hinge 29. The door hinge 29 is located at the edge defined between the front door 26 and one of the two side walls 22. It is evident that, in other embodiments, the front door 26 may be rolling, folded (e.g., bi-folded), sliding, etc. Further preferably, the front door 26 includes a handle 30 for opening/closing the front door 26. The handle 26 projects from the front door 26 in a direction opposite the chamber 21 in a way that the handle 26 is easily manoeuvrable by a user from the outside. In one embodiment, the front wall 20a comprises a control panel. One or more buttons 20b are provided on the control panel of the front wall 20a to set and manage the irradiation process carried out by the apparatus 20. Advantageously, the control panel of the front wall 20a further includes a display 20c to monitor the irradiation process. Further advantageously, the display 20c is a touch screen display. In one embodiment, the display 20c is substantially flat and has a square shape. Advantageously, the display 20c is coplanar with or parallel to the front wall 20a.

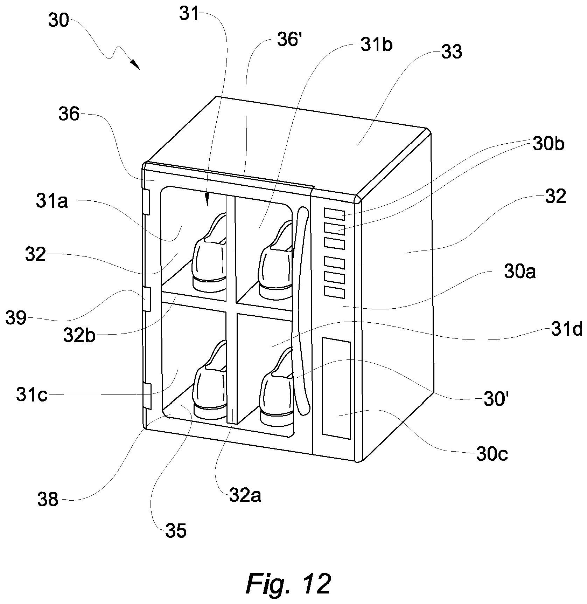

Further, with now reference to fig. 12, an apparatus 30 for the irradiation of a space, surface or object according to the method of the invention is depicted. The apparatus 30 is a box device. The apparatus 30 comprises a chamber 31. In one embodiment, the apparatus 30 has a box enclosure structure and the chamber 31 is defined by a plurality of walls 32, 33, 34, 35 of said box enclosure structure. Preferably, the walls of the plurality of walls 32, 33, 34, 35 are substantially flat and define a parallelepiped shape of the box enclosure structure and of the chamber 31. In particular, the box enclosure structure includes a front wall 30a, two opposite side walls 32, a top wall 33, a back wall (not visible), and a bottom wall 35. Preferably, the box enclosure structure further includes a front opening 36' through the front wall 30a for inserting the object into the chamber 31 and for extracting the object from inside the chamber 31. Further preferably, the box enclosure structure includes a front door 36 to close the front opening 36'. The front door 36 is coplanar with the front wall 30a. The chamber 31 is split into four sub-chambers 31a, 31b, 31c, 31d by a first and a second mutually crossed partition walls 32a and 32b located inside the chamber 31. Preferably, the first partition wall 32a is parallel to the side walls 32 and the second partition wall 32b is parallel to the top and bottom walls 33 and 35. Advantageously, the first partition wall 32a allows to support the weight of one or more objects inserted into the sub-chambers 31a, 31b. Each of the sub-chambers 31a, 31b, 31c, 31d is configured to receive the object (such as for example a shoe or a pair of shoes). At least one panel 1 (not visible) is provided to irradiate the object inside each of the sub-chambers 31a, 31b, 31c, 31d and/or a surface of the object inside each of the sub-chambers 31a, 31b, 31c, 31d and/or the space inside each of the sub-chambers 31a, 31b, 31c, 31d. Advantageously, the apparatus 30 is configured to disinfect or sanitize the object or the surface of the object inserted into any of the sub-chambers 31a, 31b, 31c, 31d. The panel(s) 1 may be mounted to a side wall 32 and/or a partition wall 32a, 32b and/or the top wall 33 and/or the bottom wall 35 and/or the back wall of the chamber 31 in a way that the optical element(s) of the panel(s) 1 emit the electromagnetic radiation toward the object(s) inserted into the chamber 31. In one embodiment, the front door 36 has a front window 38 made of a material which is transparent to visible electromagnetic radiation to allow a user to monitor the chamber 31 from the outside. The front window 38 is substantially flat and coplanar with the front door 36. Preferably, the front door 36 is articulated to the box enclosure structure by means of a door hinge 39. The door hinge 39 is located at the edge defined between the front door 36 and one of the two side walls 32. It is evident that, in other embodiments, the front door 36 may be rolling, folded (e.g., bi-folded), sliding, etc. Further preferably, the front door 36 includes a handle 30' for opening/closing the front door 36. The handle 30' projects from the front door 36 in a direction opposite the chamber 31 in a way that the handle 30' is easily manoeuvrable by a user from the outside. In one embodiment, the front wall 30a comprises a control panel. One or more buttons 30b are provided on the control panel of the front wall 30a to set and manage the irradiation process carried out by the apparatus 30. Advantageously, the control panel of the front wall 30a further includes a display 30c allows to monitor the irradiation process. Further advantageously, the display 30c is a touch screen display. In one embodiment, the display 30c is substantially flat and has a square shape. Advantageously, the display 30c is coplanar with or parallel to the front wall 30a.

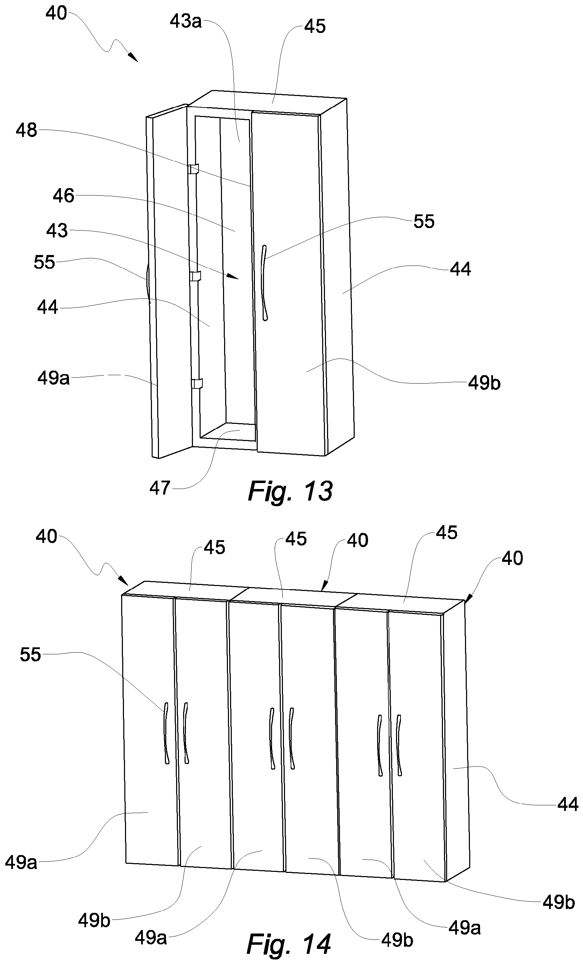

Further, with now reference to fig. 13, an apparatus 40 for the irradiation of a space, surface or object according to the method of the invention is depicted. The apparatus 40 is a piece of furniture such as for example a closet. The apparatus 40 comprises a chamber 43 configured to receive the object (such as for example a garment), and at least one panel 1 (not visible) configured to irradiate the object inside the chamber 43 and/or a surface of the object inside the chamber 43 and/or the volume of the chamber 43. Advantageously, the apparatus 40 is configured to disinfect or sanitize the object inserted into the chamber 43. The apparatus 40 is provided with two opposite side walls 44, a top wall 45, a back wall 46, and a bottom wall 47 defining the chamber 43. Preferably, the chamber 43 is split in two sub-chambers by a partition wall 48 (only one sub-chamber 43a of the two sub-chambers is visible in Fig. 13). Further preferably, each sub-chamber is equipped with at least one panel 1 for the application of the method according to the present invention. The panel(s) 1 may be mounted to a side wall 44 and/or the partition wall 48 and/or the top wall 45 and/or the bottom wall 47 and/or the back wall 46 in a way that the optical element(s) of the panel(s) 1 emit the electromagnetic radiation toward the object(s) inserted into the chamber 31 and/or toward the object(s) inserted into the sub-chambers of the chamber 43. In one embodiment, the apparatus 40 is provided with two front doors 49a, 49b. Preferably, the front doors 49a, 49b are located in front of the sub-chambers of the chamber 43. Each front door 49a, 49b may be movable between a closed position and an open position. In the closed position, each front door 49a, 49b prevents radiation within the relevant sub-chamber from exiting the sub-chamber and affecting surrounding objects and/or persons. In the open position, each front door 49a, 49b enables an object to be positioned within and/or removed from the relevant sub-chamber. Preferably, each front door 49a, 49b includes a handle 55 for opening/closing the respective front door 49a, 49b. In one embodiment, further partition walls can be added in order to split the chamber 43 into the desired number of sub-chambers. Preferably, the number of sub-chambers is comprised between 2 and 12.

Further, with now reference to fig. 14, the apparatus 40 has a modular design which allows to arrange a plurality of apparatuses 40 adjacent one each other. Preferably, the apparatuses 40 may be arranged adjacent one each other along their side walls 44. In one embodiment, three apparatuses 40 are arranged adjacent one each other along their side walls 44. Advantageously, the top walls 45 of the apparatuses 40 arranged together are coplanar. Further advantageously, the front doors 49a, 49b of the apparatuses 40 arranged together are all directed alike.

The present invention will be now further illustrated from the following examples.

### Examples

### Example 1

### Effect of the blue light in bacteria

The Applicant have performed studies to understand the effect that the blue light, at the claimed intensity and for expositions in the claimed time range, has on the activity of some Bacteria species.

### Materials and methods

Measurements for cell viability have been used.

The most direct means of measuring cell proliferation, a determination of the number of actively dividing cells, is to count the number of cells present. Cell viability, defined as the number of metabolically active cells in a sample, determines the amount of cells (regardless of phase around the cell cycle) that are living or dead, based on a total cell sample.

Tests have been performed on the following bacteria: Escherichia Coli NCTC 12923; Staphylococcus Aureus. NCTC 10788; Pseudomonas Aeruginosa NCTC 12924.

A number of plates with known bacteria concentration has been irradiated with pulsed (400 KHz repetition rate) blue light at different power and energy intensities for time intervals of 20, 60 or 180 minutes and subsequently incubated at suitable temperatures. The bacteria have been also observed after 24h and 48h from the first irradiation

### Results

A substantial and constant bacteria reduction has been observed ranging from a minimum of 48% to a maximum of 100% effectiveness, as shown in the following Table 3.

**Table 3**

| Bacteria Type | Power density mW/cm² | Time (minutes) | Energy density J/cm² | Reduction % |
|---|---|---|---|---|
| Escherichia Coli NCTC 12923 | 18 | 180 | 200 | 93,5 |
| | 16 | 180 | 175 | 65,26 |
| | 13 | 180 | 140 | 57,69 |
| | 51 | 60 | 185 | 100 |
| | 51 | 20 | 61 | 49,1 |
| Staphilococcus A. NCTC 10788 | 18 | 180 | 200 | 86,07 |
| | 16 | 180 | 175 | 72,1 |
| | 13 | 180 | 140 | 49,75 |
| | 51 | 60 | 185 | 92,2 |
| Pseudomonas A. NCTC 12924 | 51 | 60 | 185 | 99,96 |
| | 51 | 20 | 61 | 66,8 |

From the data collected by the Applicant, it is clear that most of the bacteria, if irradiated with pulsed blue light for a duration of between 20 minutes and 3 hours and with an energy density comprised between 61 J/cm² and 200 J/cm² are killed.

### Example 2

### Effect of the blue light on virus activity

Tests have been performed on: Adenovirus (ADV Pondicherry strain/JIPMER/60), Respiratory Syncytial Virus (RSV type A strain long ATCC VR-26) and SARS-COV-2 (GeneBank accession no. MT531537).

### Materials and methods

### Respiratory Syncytial Virus (RSV)

Respiratory Syncytial Virus 9.3x10⁵ TCID₅₀/ml VeroE6(3) was used.

Experiments were performed with a 200 mW and a 70 mW lamp.

1 ml of virus was placed in a petri dish with a diameter of 4 cm and left under a lamp of 70 or 120 mW without cover for 30 minutes under Biohazard hood class 2. Since part of the liquid was evaporated, the remaining volume was of 900 µl. The volume was taken to 1 ml with Dulbecco's modified eagle medium (D-MEM) (SS) and the virus was immediately titled diluting in base 10 from 10⁻¹ to 10⁻⁸ on virus sensitive VERO E6 cells (ATCC CRL-1586).

For the evaluation of the viral title 1 ml of virus was placed in the same conditions of the treated virus in the absence of the lamp (control).

For the titration, dilutions in base 10 in DMEM (SS) of the treated virus were carried out. 50 µl of the dilutions were plated in quadruplicate in 96 wells platelets. 50 µl of VERO E6 cells (ATCC CRL-1586) were added in each well (2 x 10⁵ cells/ml) and the plate was incubated at 37°C at 5% CO₂ in incubator (Heraeus, Hera cell 240). After 4 days control of the cells was carried out at the optical microscope for the search of the cytopathic effect (CPE). The viral title was calculated based on the formula of "Reed and Muench" (Reed U, Muench H. A simple method of estimating fifty per cent endpoints. Am J Hyg. 1938;27:493-497).

### Adenovirus (ADV)

Adenovirus pure title >10⁹ TCID₅₀/Ml VeroE6(2) was used.

Experiments were performed with a 200 mW and a 70 mW lamp.

1 ml of virus was placed in a petri dish with a diameter of 4 cm and left under the lamp without cover for 30 minutes under Biohazard hood class 2. Since part of the liquid was evaporated, the remaining volume was of 900 µl. The volume was taken to 1 ml with D-MEM (SS) and the virus was immediately titled diluting in base 10 from 10⁻¹ to 10⁻⁸ on virus sensitive VERO E6 cells (ATCC CRL-1586).

For the evaluation of the viral title 1 ml of virus was placed in the same conditions of the treated virus in the absence of the lamp (control).

For the titration, dilutions in base 10 in DMEM (SS) of the treated virus were carried out. 50 µl of the dilutions were plated in quadruplicate in 96 wells platelets. 50 µl of VERO E6 cells (ATCC CRL-1586) were added in each well (2 x 10⁵ cells/ml) and the plate was incubated at 37°C at 5% CO₂ in incubator (Heraeus, Hera cell 240). After 3 days control of the cells was carried out at the optical microscope for the search of the cytopathic effect (CPE). The viral title was calculated based on the formula of "Reed and Muench".

### SARS-COV-2

SARS-COV-2 pure title 6.3x10⁴/ml VeroE6(2) was used.

Experiments were performed with a 200 mW lamp.

1 ml of virus was placed in a petri dish with a diameter of 4 cm and left under the lamp without cover for 15 or 30 minutes under Biohazard hood class 2. Since part of the liquid was evaporated, the remaining volume was of 900 µl. The volume was taken to 1 ml with D-MEM (SS) and the virus was immediately titled diluting in base 10 from 10⁻¹ to 10⁻⁸ on virus sensitive VERO E6 cells (ATCC CRL-1586).

For the evaluation of the viral title 1 ml of virus was placed in the same conditions of the treated virus in the absence of the lamp (control).

For the titration, dilutions in base 10 in DMEM (SS) of the treated virus were carried out. 50 µl of the dilutions were plated in quadruplicate in 96 wells platelets. 50 µl of VERO E6 cells (ATCC CRL-1586) were added in each well (2 x 10⁵ cells/ml) and the plate was incubated at 37°C at 5% CO₂ in incubator (Heraeus, Hera cell 240). After 4 days control of the cells was carried out at the optical microscope for the search of the cytopathic effect (CPE). The viral title was calculated based on the formula of "Reed and Muench".

### Results

A number of vials with known virus concentration has been irradiated with pulsed (400 KHz) blue light at different quantity of energy intensity. The viruses have been then observed after 72h and a substantial reduction have been observed ranging from a minimum of 99,0% to a maximum of 100% effectiveness, as shown in the following Table 4.

**Table 4**

| Virus Type | Power mW/cm² | Time (minutes) | Treatment intensity J/cm² | Reduction % |
|---|---|---|---|---|
| ADV | 120 | 30 | 216 | 100 |
| | 70 | 30 | 126 | 99,9 |
| RSV | 120 | 30 | 216 | 100 |
| COVID-19 | 120 | 30 | 216 | 100 |
| | 120 | 15 | 108 | 100 |

Therefore, the process of the invention using electromagnetic radiation destroys bacteria and viruses *in vitro.* These modifications, without being bound by any theory, might be the reason of the disinfection or sanitization.

## Claims

1. A method for inactivation of viruses and/or bacteria of at least 50% in a space, a surface or an object comprising irradiating said space, surface or object with an electromagnetic radiation having a wavelength comprised between 410 nm and 430 nm for a time interval comprised between 5 minutes and 180 minutes, with a power density comprised between 13 mW/cm² and 120 mW/cm² and an energy density comprised between 30 J/cm² and 220 J/cm², wherein the electromagnetic radiation is irradiated at a distance between the source of the electromagnetic radiation and the irradiated space, surface or object comprised from 30 centimetres to 1,5 meter.

2. The method according to claim 1 which is for inactivation of bacteria of at least 50% in a space, a surface or an object and comprises irradiating said space, surface or object with an electromagnetic radiation having a wavelength comprised between 410 nm and 430 nm for a time interval comprised between 20 minutes and 180 minutes with a power density comprised between 13 mW/cm² and 120 mW/cm² and an energy density comprised between 60 J/cm² and 200 J/cm², wherein the electromagnetic radiation is irradiated at a distance between the source of the electromagnetic radiation and the irradiated space, surface or object comprised from 30 centimetres to 1,5 meter.

3. The method according to claim 2, wherein the electromagnetic radiation is applied for a time interval comprised between 20 minutes and 60 minutes, with a power density comprised between 18 mW/cm² and 60 mW/cm² and an energy density comprised between 140 J/cm² and 185 J/cm².

4. The method according to claim 3, wherein the electromagnetic radiation is applied for a time interval of 180 minutes, with a power density of 18 mW/cm² and an energy density of 200 J/cm²; or it is applied for a time interval of 60 minutes, with a power density of 51mW/cm² and an energy density of 185 J/cm².

5. The method according to claim 1 which is for inactivation of viruses of at least 50% in a space, a surface or an object comprising irradiating said space, surface or object with an a electromagnetic radiation having a wavelength comprised between 410 nm and 430 nm for a time interval comprised between 5 minutes and 30 minutes with a power density comprised between 70 mW/cm² and 120 mW/cm² and an energy density comprised between 30 J/cm² and 220 J/cm², wherein the electromagnetic radiation is irradiated at a distance between the source of the electromagnetic radiation and the irradiated space, surface or object comprised from 30 centimetres to 1,5 meter.

6. The method according to claim 5, wherein the electromagnetic radiation is applied for a time interval comprised between 15 and 30 minutes, with a power density comprised between 70 mW/cm² and 120 mW/cm² and an energy density comprised between 108 J/cm² and 216 J/cm².

7. The method according to claim 6, wherein the electromagnetic radiation is applied for a time interval of 30 minutes, with a power density of 120 mW/cm² and an energy density of 216 J/cm²; or it is applied for a time interval of 30 minutes, with a power density of 70 mW/cm² and an energy density of 126 J/cm²; or it is applied for a time interval of 15 minutes, with a power density of 120 mW/cm² and an energy density of 108 J/cm².

8. The method according to anyone of claims 1-4, wherein the method is for disinfecting or sanitizing the irradiated space, surface or object from bacteria of one or more species selected from the group consisting of *Escherichia Coli, Staphilococcus Aureus* and *Pseudomonas Aeruginosa.*

9. The method according to anyone of claims 5-7, wherein the method is for disinfecting or sanitizing the irradiated space, surface or object from viruses belonging to one or more virus families selected from the group consisting of: adenovirus, Respiratory Syncytial Virus and coronavirus.

10. The method according to claim 9, wherein the virus is a coronavirus of the strain severe acute respiratory syndrome coronavirus 2 (SARS-CoV-2), causing the coronavirus disease 19 (COVID-19).

11. The method according to anyone of the preceding claims, wherein a single irradiation of said space, surface or object is performed.

12. The method according to anyone of the preceding claims, wherein the irradiation is performed using one or more LEDs, preferably an array of LEDs, wherein the number of LEDs is preferably comprised between 1 and 36.

13. The method according to anyone of the preceding claims, wherein the irradiation is performed using an apparatus selected from a portable apparatus, a free standing apparatus, a table standing apparatus and a whole case enclosure apparatus.

14. The method according to claim 13, wherein said apparatus comprise modular optics wherein each module includes a portion based on single optic panels which are assembled in multiples of 1 to 8.

15. The method according to one or more of the preceding claims, which is adapted for the disinfection or sanitization of a space, a surface or an object from viruses and/or bacteria.

16. The method according to one or more of the preceding claims, wherein the electromagnetic radiation is a pulsed electromagnetic radiation having a pulse repetition comprised between 10 KHz and 400 KHz.

17. The method according to one or more of the preceding claims, wherein the distance between the source of the electromagnetic radiation and the irradiated space, surface or object is comprised from 30 centimetres to 1 meter.
